# EUROPEAN PATENT APPLICATION

(11) **EP 4 524 980 A1**
(43) Date of publication of application: **19.03.2025**
(21) Application number: 23846049.7
(22) Date of filing: 14.06.2023
(51) Int. Cl.: G16H 20/00

(54) **COMPUTER PROGRAM, INFORMATION PROCESSING DEVICE, INFORMATION PROCESSING METHOD, AND LEARNING MODEL GENERATION METHOD**

(30) Priority: 25.07.2022 JP 2022118143
(71) Applicant: Terumo Kabushiki Kaisha, Tokyo 151-0072 (JP)
(72) Inventor: SHIMADA, Naoya, Ashigarakami-gun, Kanagawa 259-0151 (JP); KIKUCHI, Hideka, Ashigarakami-gun, Kanagawa 259-0151 (JP); YAGO, Seiji, Ashigarakami-gun, Kanagawa 259-0151 (JP); MARUYAMA, Tomoji, Ashigarakami-gun, Kanagawa 259-0151 (JP); TAKEUCHI, Takanori, Tokyo 163-1450 (JP); YOKOUCHI, Atsushi, Tokyo 163-1450 (JP); FUJII, Naoko, Ashigarakami-gun, Kanagawa 259-0151 (JP)
(74) Representative: KIPA AB
(86) International application number: PCT/JP2023/022147
(87) International publication number: WO 2024/024315

(57) **Abstract**

A computer program, an information processing device, an information processing method, and a learning model generation method that assist appropriate pain management are provided.

The computer program causes a computer to execute processing including acquiring pain related information regarding pain of a patient, in a case where the pain related information is input, inputting the acquired pain related information into a learning model that outputs pain prediction information and acquiring the pain prediction information of the patient, generating pain treatment assistance information for the patient based on the acquired pain prediction information, and outputting the generated pain treatment assistance information.

## Description

### Technical Field

The present invention relates to a computer program, an information processing device, an information processing method, and a learning model generation method.

### Background Art

Patent Literature 1 discloses a medical information management system that includes a plurality of medical department servers that provides medical information stored in a database of a plurality of medical departments in a medical institution and a specific disease treatment integrated server that acquires specific disease medical information from the medical department server and provides the integrated specific disease medical information from an integrated database, in which a treatment progressing status can be grasped with high immediacy by making it possible to refer to medical information of specific diseases such as cancer in all treatment methods combining a surgery, radiation therapy, chemotherapy, and palliative care and registered related information of the specific diseases in a wide range.

### Citation List

### Patent Literature

Patent Literature 1: JP 2010-3135 A

### Summary of Invention

### Technical Problem

For diseases such as cancer, there is a method of short-term prognosis prediction (for example, palliative prognostic index (PPI)). However, this is prognosis regarding a medical condition and is not specialized in pain caused by the disease. Furthermore, there is a problem that a doctor can evaluate pain only when the doctor faces a patient. Furthermore, specialized knowledge is essential for effective pain management, and there is a problem that it is difficult for a doctor other than a specialist to perform an accurate pain treatment according to the patient.

The present invention has been made in view of the above circumstances, and an object of the present invention is to provide a computer program, an information processing device, an information processing method, and a learning model generation method that can assist an appropriate pain treatment.

### Solution to Problem

(1) A computer program according to the present invention causes a computer to execute processing including acquiring pain related information regarding pain of a patient, in a case where the pain related information is input, inputting the acquired pain related information into a learning model that outputs pain prediction information and acquiring the pain prediction information of the patient, generating pain treatment assistance information for the patient based on the acquired pain prediction information, and outputting the generated pain treatment assistance information.
   Here, in an embodiment of the present invention,
(2) in the computer program according to (1), the pain related information preferably includes at least one of an evaluation index used to evaluate pain, vital data, medication information, and a physical condition.
(3) The computer program according to (1) or (2), preferably causes the computer to execute processing further including acquiring medical information regarding medical care of the patient, and in a case where the medical information is further input, inputting the acquired medical information into the learning model that outputs the pain prediction information of the patient and acquiring the pain prediction information of the patient.
(4) The computer program according to any one of (1) to (3), in which the medical information preferably includes at least one of diagnosis information, examination information, treatment information, prescription information, and disease prognosis prediction information.
(5) The computer program according to any one of (1) to (4), preferably causes the computer to execute processing further including acquiring literature information regarding a pain treatment, and in a case where the literature information regarding the pain treatment is further input, inputting the acquired literature information into the learning model that outputs the pain prediction information and acquiring the pain prediction information of the patient.
(6) The computer program according to any one of (1) to (5), in which the pain treatment assistance information preferably includes at least one of recommended prescription information and recommended treatment information used to assist a doctor who examines the patient.
(7) The computer program according to any one of (1) to (6), in which the pain treatment assistance information preferably includes a coping method for assisting the patient.
(8) The computer program according to any one of (1) to (7), preferably causes the computer to execute processing including outputting a pain prediction pattern indicating a temporal transition of a degree of pain based on the pain prediction information of the patient.
(9) An information processing device according to the present invention includes a first acquisition unit that acquires pain related information regarding pain of a patient, a second acquisition unit that inputs the acquired pain related information into a learning model that outputs pain prediction information in a case where the pain related information is input and acquires the pain prediction information of the patient, a generation unit that generates pain treatment assistance information for the patient based on the acquired pain prediction information, and an output unit that outputs the generated pain treatment assistance information.
(10) An information processing method according to the present invention includes acquiring pain related information regarding pain of a patient, in a case where the pain related information is input, inputting the acquired pain related information into a learning model that outputs pain prediction information and acquiring the pain prediction information of the patient, generating pain treatment assistance information for the patient based on the acquired pain prediction information, and outputting the generated pain treatment assistance information.
(11) A learning model generation method according to the present invention includes acquiring first training data including pain related information regarding pain of a plurality of patients and pain prediction information of the plurality of patients and generating a learning model so as to output the pain prediction information in a case where the pain related information is input, based on the acquired first training data.
(12) The learning model generation method according to (11), in the learning model generation method according to supplementary note 11, preferably includes acquiring second training data further including medical information of the plurality of patients, literature information regarding a pain treatment, and the pain prediction information of the plurality of patients and generating the learning model so as to output the pain prediction information, in a case where the medical information and the literature information regarding the pain treatment are input, based on the acquired second training data.

### Advantageous Effects of Invention

According to the present invention, an appropriate pain treatment can be assisted.

### Brief Description of Drawings

Fig. 1 is a diagram illustrating an example of a configuration of a pain treatment assistance system according to the present embodiment.
Fig. 2 is a diagram illustrating an example of a configuration of a pain treatment assistance device.
Fig. 3 is a diagram illustrating information collection by the pain treatment assistance device.
Fig. 4A is a diagram illustrating an example of information in a database.
Fig. 4B is a diagram illustrating an example of the information in the database.
Fig. 4C is a diagram illustrating an example of the information in the database.
Fig. 5 is a diagram illustrating provision of pain treatment assistance information by the pain treatment assistance device.
Fig. 6 is a diagram illustrating a first example of processing for generating the pain treatment assistance information by the pain treatment assistance device.
Fig. 7 is a diagram illustrating a second example of the processing for generating the pain treatment assistance information by the pain treatment assistance device.
Fig. 8 is a diagram illustrating a third example of the processing for generating the pain treatment assistance information by the pain treatment assistance device.
Fig. 9 is a diagram illustrating an example of a learning model generation method.
Fig. 10 is a diagram illustrating a first example of pain treatment assistance information on a doctor side.
Fig. 11 is a diagram illustrating a third example of the pain treatment assistance information on the doctor side.
Fig. 12 is a diagram illustrating a third example of the pain treatment assistance information on the doctor side.
Fig. 13 is a diagram illustrating a fourth example of the pain treatment assistance information on the doctor side.
Fig. 14 is a diagram illustrating a first example of the pain treatment assistance information on a patient side.
Fig. 15 is a diagram illustrating a second example of the pain treatment assistance information on the patient side.
Fig. 16 is a diagram illustrating an example of a processing procedure by the pain treatment assistance device.
Fig. 17 is a diagram illustrating an example of a learning model generation procedure.

### Description of Embodiments

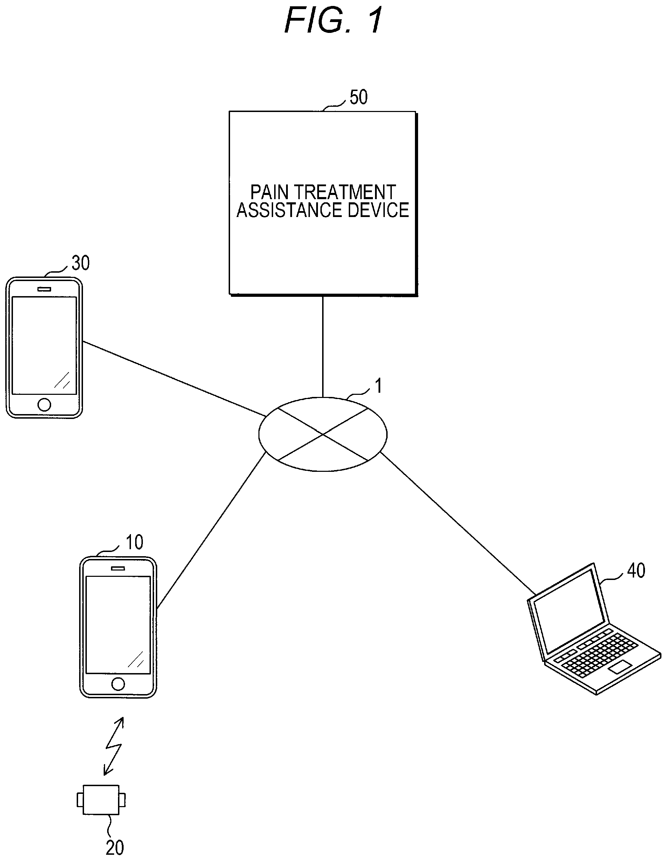

Hereinafter, an embodiment of the present invention will be described. Fig. 1 is a diagram illustrating an example of a configuration of a pain treatment assistance system according to the present embodiment. The pain treatment assistance system as an information processing system includes a pain treatment assistance device 50 as an information processing device. The pain treatment assistance system may include a patient terminal device 10, a device 20, an assistant terminal device 30, and a doctor terminal device 40. The patient terminal device 10, the assistant terminal device 30, and the doctor terminal device 40 are connected to the pain treatment assistance device 50 via a communication network 1. The device 20 may have a configuration that is wirelessly or wiredly connected to the patient terminal device 10 or may have a configuration that is connected to the pain treatment assistance device 50 via the communication network 1. Note that the device 20 is not essential, and for example, a configuration may include only input from the patient terminal device 10.

The patient terminal device 10 is a terminal device carried or held by a patient, and the assistant terminal device 30 is a terminal device carried or held by an assistant such as a family member or a caregiver of the patient. The caregiver may be a family member or a caregiver at a care provider. The patient terminal device 10 and the assistant terminal device 30 can include a smartphone, a tablet, a personal computer, or the like including a display panel, an operation panel, a microphone, a speaker, or the like. In the patient terminal device 10 and the assistant terminal device 30, an app (application) for using the pain treatment assistance system is introduced. For example, a patient who is suffering from cancer or the like and periodically visits a medical institution while being treated at home is assumed. However, the patient is not limited to such a patient. Note that the app may be a Web app or a downloaded app.

The device 20 is a measuring instrument that measures vital data of the patient and may be a wearable terminal or a stationary type. The device 20 can measure the vital data such as a body temperature, a heart rate, brain waves, or electrodermal activity (EDA). Note that the vital data is not limited to these, and may include any data assumed to be related to pain caused by the patient's disease. For example, a blood pressure, a pulse pressure, pulse waves, a blood sugar level, a weight, the number of steps, an activity amount, or the like may be included in the vital data.

The doctor terminal device 40 is a terminal device used by a doctor in a medical institution or the like. The doctor terminal device 40 can include a personal computer or the like including a display panel, an operation panel, or the like. A medical worker such as a public health nurse or a nurse may use the doctor terminal device 40 under guidance of the doctor. An application (for example, Web app or the like) for using the pain treatment assistance system is introduced in the doctor terminal device 40.

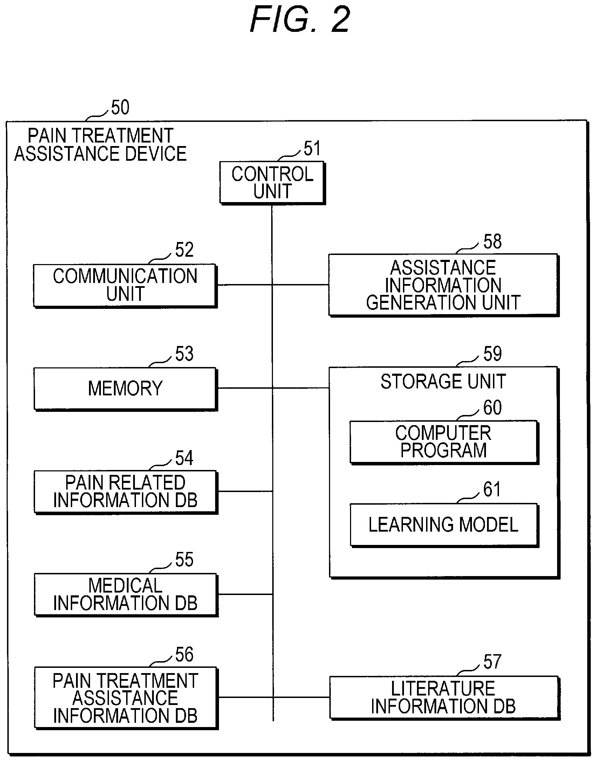

Fig. 2 is a diagram illustrating an example of a configuration of the pain treatment assistance device 50. The pain treatment assistance device 50 includes a control unit 51 that controls an entire device, a communication unit 52, a memory 53, a pain related information DB 54, a medical information DB 55, a pain treatment assistance information DB 56, a literature information DB 57, an assistance information generation unit (generation unit) 58, and a storage unit 59. The pain treatment assistance device 50 may distribute processing functions and include a plurality of devices. A database such as the pain related information DB 54, the medical information DB 55, the pain treatment assistance information DB 56, or the literature information DB 57 may be built in the pain treatment assistance device 50 or may be incorporated into an external data server. Furthermore, the storage unit 59 may be incorporated into an external data server.

The storage unit 59 can include, for example, a hard disk, a semiconductor memory, or the like, and stores a computer program 60 (program product), a learning model 61, and necessary information. The computer program 60 may be downloaded from an external device via the communication unit 52 and stored in the storage unit 59. Furthermore, the computer program 60 recorded in a recording medium (for example, optically readable disk storage medium such as CD-ROM) may be read by a recording medium reading unit and stored in the storage unit 59, or the computer program 60 may be read by the recording medium reading unit and developed in the memory 53.

The control unit 51 may be configured by incorporating a required number of central processing units (CPUs), micro-processing units (MPUs), graphics processing units (GPUs), or the like. The control unit 51 can execute processing defined by the computer program 60. That is, the processing executed by the control unit 51 corresponds to processing executed in accordance with the computer program 60. The control unit 51 can execute functions of the assistance information generation unit 58, by executing the computer program 60. The assistance information generation unit 58 may include hardware or software or may be implemented by a combination of hardware and software. The control unit 51 can execute processing using the learning model 61. Note that the control unit 51 has functions as a first acquisition unit, a second acquisition unit, a generation unit, and an output unit.

The communication unit 52 includes a communication module and can exchange necessary information with the patient terminal device 10, the assistant terminal device 30, and the doctor terminal device 40 via the communication network 1. Furthermore, the communication unit 52 may exchange necessary information with the device 20.

The memory 53 may include a semiconductor memory, such as a static random access memory (SRAM), a dynamic random access memory (DRAM), or a flash memory. Loading the computer program 60 into the memory 53 enables the control unit 51 to execute the computer program 60.

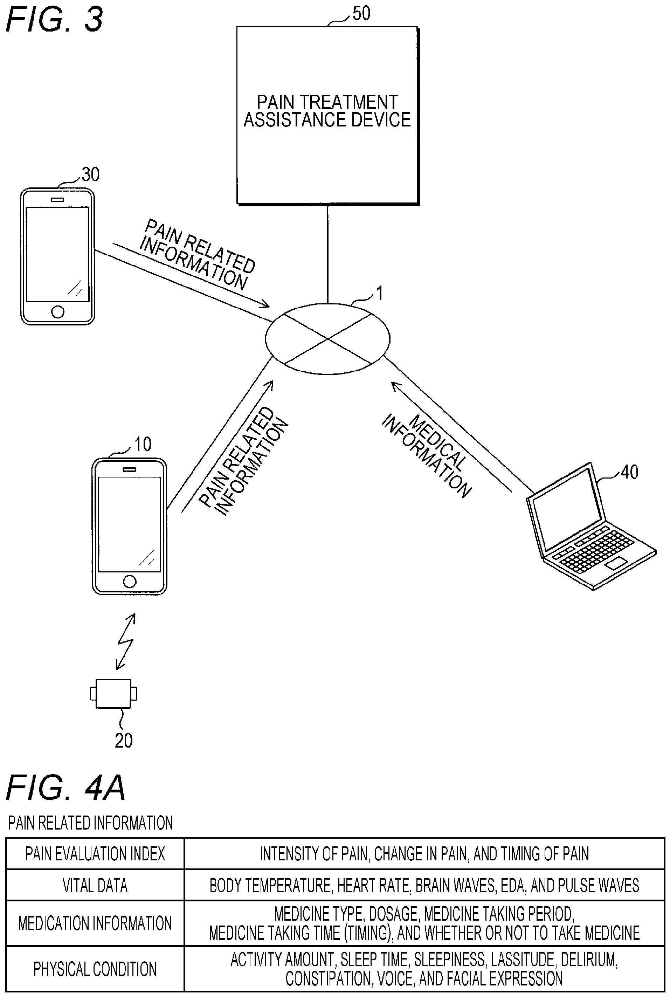

Fig. 3 is a diagram illustrating information collection by a pain management assistance device 50. The pain management assistance device 50 acquires pain related information related to pain caused by a patient's disease from the patient terminal device 10, the device 20, and the assistant terminal device 30 via the communication unit 52. The pain related information is repeatedly transmitted from the patient terminal device 10, the device 20, and the assistant terminal device 30 as appropriate. For example, the patient may transmit the pain related information once in a period from a previous hospital visit to a next hospital visit or may transmit the pain related information a plurality of times according to a length of the period. The acquired pain related information is classified for each patient and for each acquired time point and stored in the pain related information DB 54.

Furthermore, the pain treatment assistance device 50 acquires medical information related to medical care of the patient from the doctor terminal device 40 via the communication unit 52. The acquired medical information is classified for each patient and for each medical care time point and stored in the medical information DB 55.

Figs. 4A to 4C are diagrams illustrating an example of information in a database. Fig. 4A illustrates an example of information in the pain related information DB 54. The pain related information can be classified into a pain evaluation index (evaluation index used to evaluate pain), vital data, medication information, and a physical condition. The pain related information transmitted from the patient terminal device 10 and the device 20 includes at least one of the pain evaluation index, the vital data, the medication information, and the physical condition. Note that the classification is an example and is not limited to the example in Fig. 4A.

The pain evaluation index includes an intensity of pain, a change in the pain, a timing of the pain, or the like. The pain evaluation index can be transmitted from the patient terminal device 10 to the pain treatment assistance device 50, for example, when the patient activates the app of the patient terminal device 10 and answers a question regarding the intensity of the pain, the change in the pain, the timing of the pain, or the like.

The vital data includes the body temperature, the heart rate, the brain waves, the EDA, the pulse waves, or the like that changes in response to the pain. Note that the blood pressure, the pulse pressure, the blood sugar level, the weight, the number of steps, the activity amount, or the like may be included. The vital data can be acquired using the device 20.

The medication information includes a medicine type, a dosage, a medicine taking period, a medicine taking time (timing), whether or not to take the medicine, or the like. The medication information may include not only a medicine that is currently taken but also a medicine that has been taken in the past.

The physical condition includes an activity amount, a sleep time, sleepiness, lassitude, delirium, constipation, voice, facial expression, or the like. It is possible to include an item that can evaluate whether or not a disorder occurs due to the pain, the medicine, medical conditions, or the like.

The pain related information includes at least one of the pain evaluation index (evaluation index used to evaluate pain), the vital data, the medication information, and the physical condition.

By acquiring the pain related information as described above from the patient terminal device 10 and the device 20, the patient can accurately tell pain and symptoms that the patient feels at a necessary timing and a necessary frequency, in a period from a previous examination to a next examination.

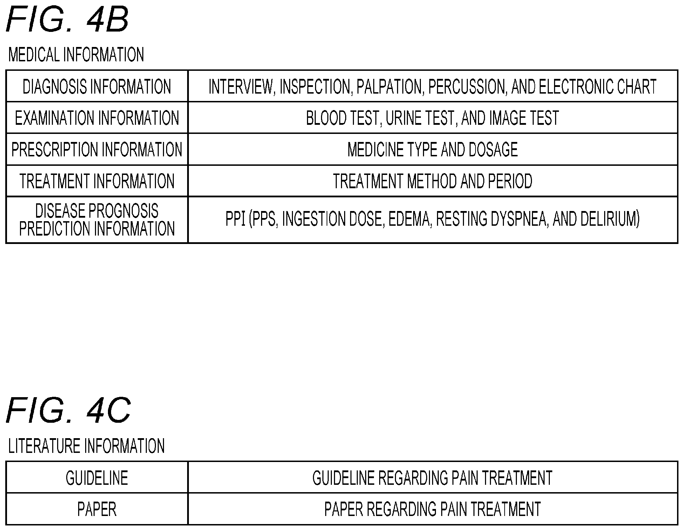

Fig. 4B illustrates an example of information in the medical information DB 55. The medical information can be classified into diagnosis information, examination information, prescription information, treatment information, and disease prognosis prediction information. The medical information transmitted from the doctor terminal device 40 includes at least one of the diagnosis information, the examination information, the prescription information, the treatment information, and the disease prognosis prediction information. Note that the classification is an example and is not limited to the example in Fig. 4B.

The diagnosis information includes findings by interview, inspection, palpation, or percussion at the time of examination, an electronic chart, or the like. The examination information includes a blood test, a urine test, an image test, or the like. The prescription information includes a medicine type and a dosage prescribed by a doctor. Furthermore, the medicine taking period may be included. The treatment information includes a treatment method and a treatment period. Note that not only a literal treatment but also medicine prescription are included in a pain treatment. The disease prognosis prediction information includes a palliative prognostic index (PPI). The PPI is a representative index as an index used to predict a short-term life prognosis (in weeks), and a score obtained by adding scores of a palliative performance scale (PPS), an ingestion dose, an edema, resting dyspnea, and a delirium. As the score increases, predicted prognosis is shorter.

The medical information includes at least one of the diagnosis information, the examination information, the treatment information, the prescription information, and the disease prognosis prediction information.

Fig. 4C illustrates an example of information in the literature information DB 57. The literature information can be classified into a guideline and a paper. The guideline is a guideline regarding the pain treatment, and includes, for example, a guideline indicating what type of pain treatment is recommended for a symptom and pain of the patient. The paper is a paper regarding the pain treatment and includes, for example, objective information indicating what type of pain treatment is effective for the symptom and the pain of the patient. The literature information DB 57 is appropriately updated, and the latest information is recorded.

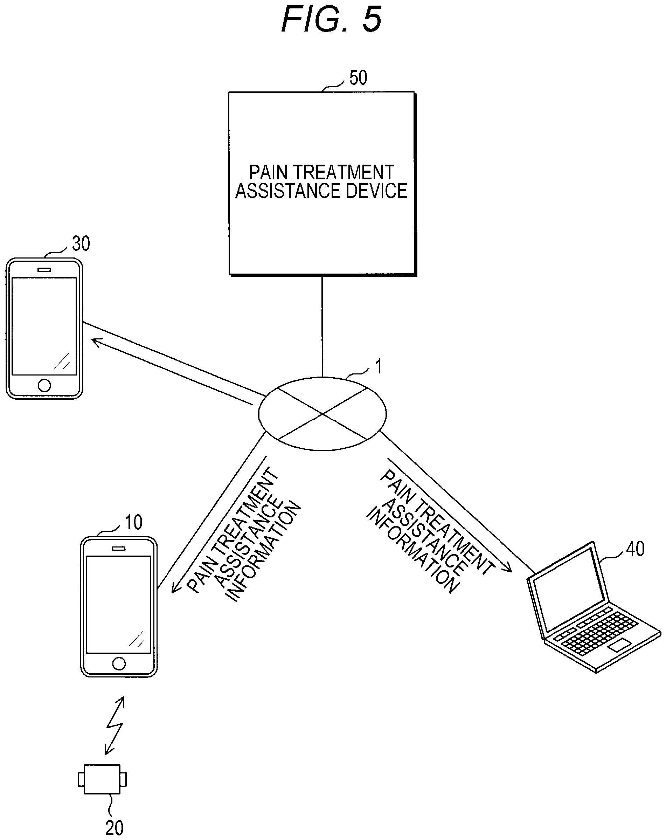

Fig. 5 is a diagram illustrating provision of pain treatment assistance information by the pain treatment assistance device 50. The pain treatment assistance device 50 (control unit 51) transmits the pain treatment assistance information generated by processing to be described later, to the patient terminal device 10, the assistant terminal device 30, and the doctor terminal device 40. A transmission timing may be a necessary timing and a necessary frequency. Note that, in the pain treatment assistance information to be provided to the patient side, information that is better to be provided to an assistant of the patient may be transmitted to the assistant terminal device 30. Furthermore, in the pain treatment assistance information to be provided to the patient side, information that is better not to be provided to the patient and to be provided only to a family member may be transmitted to the assistant terminal device 30, instead of being transmitted to the patient terminal device 10.

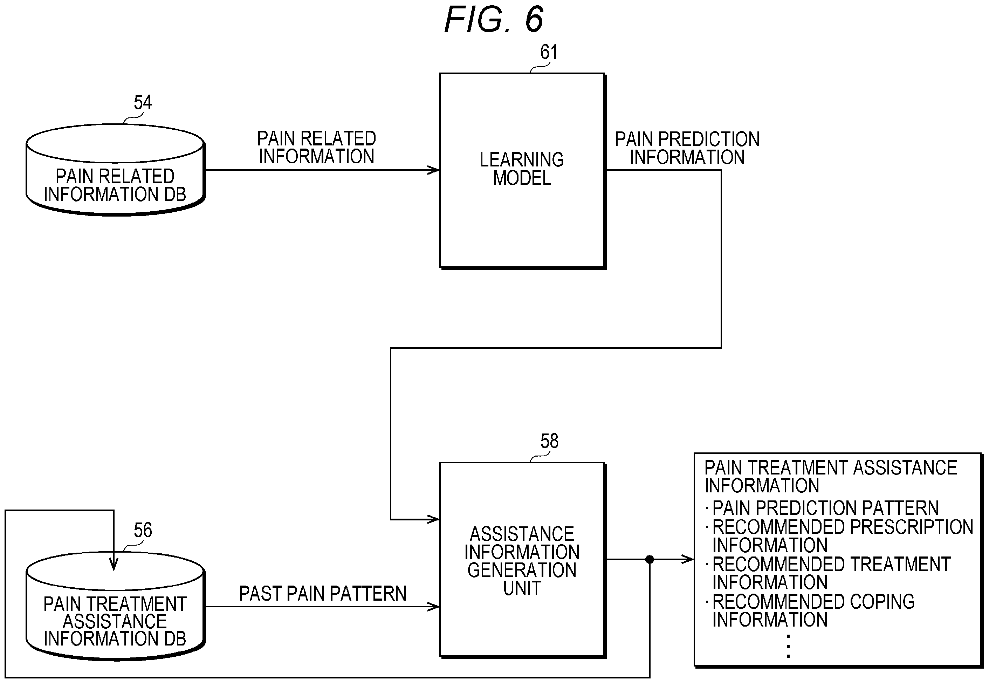

Fig. 6 is a diagram illustrating a first example of processing for generating the pain treatment assistance information by the pain treatment assistance device 50. The control unit 51 reads pain related information of a specific patient from the pain related information DB 54 and inputs the read pain related information into the learning model 61. The pain related information of the specific patient is time-series data at a single or a plurality of time points. For example, not only current pain related information of the specific patient but also pain related information at a past time point going back from the current time point may be included. The control unit 51 can generate a pain related information vector by performing a vectorization operation for generating a primary vector for the pain related information of the patient and input the generated pain related information vector into the learning model 61. Note that the pain related information vector may be generated for each of the plurality of time points. Note that, although not illustrated, a past pain pattern of the pain treatment assistance information DB 56 may be input into the learning model 61.

The learning model 61 is generated (learned) to output pain prediction information (pain prediction information) in a case where the pain related information is input. The pain related information is time-series data and includes, for example, pain related information within a period of about one month, three months, six months, or 12 months in the past going back from the present. The pain prediction information is information indicating pain within a required period from the current point to the future (for example, one month, three months, six months, 12 months, or the like). The learning model 61 may, for example, output the pain prediction information from the present to one month later based on the pain related information for past three months or may output the pain prediction information from the present to three month later, based on the pain related information for past six months. Note that these periods are examples, and the present invention is not limited to these. The degree (degree) of the pain can be expressed, for example, in several stages such as 10 stages from one to 10 or five stages from one to five. It can be assumed that the pain of the patient be stronger as a numerical value increases.

The learning model 61 can use, for example, a support vector machine (SVM), a decision tree, a random forest, an adaboost, a neural network (for example, recurrent neural network (RNN), long short term memory (LSTM), transformer, or the like), or the like.

The control unit 51 reads a past pain pattern of the patient, recorded in the pain treatment assistance information DB 56. The pain pattern is information regarding pain in the past, and is information indicating a transition of a degree of pain, similarly to the pain prediction information. A difference between the pain prediction information and the past pain pattern is that the former is a transition of a degree of pain in a period from the present to the future, whereas the latter is a transition of a degree of pain in a period from the present to the past. The control unit 51 inputs the read past pain pattern and the pain prediction information acquired from the learning model 61, into the assistance information generation unit 58.

The assistance information generation unit 58 generates the pain treatment assistance information based on the pain prediction information and the past pain pattern that have been input. The assistance information generation unit 58 stores the generated pain treatment assistance information in the pain treatment assistance information DB 56. The pain treatment assistance information includes, for example, a pain prediction pattern, recommended prescription information and recommended treatment information used to assist the doctor who examines the patient, recommended coping information used to assist the patient, or the like. It is sufficient that the pain treatment assistance information include at least one of the recommended prescription information and the recommended treatment information used to assist the doctor.

It is sufficient that the pain prediction pattern be, for example, information that is generated based on the pain prediction information and indicates a temporal transition of a degree of pain of the patient from the past to the future. The pain prediction pattern can be displayed by an appropriate method such as graph display or tabular display. The recommended prescription information is information for assisting the doctor and includes information used to determine recommended prescription **in** medicine prescription for the patient. The recommended treatment information is information for assisting the doctor and includes information used to determine a recommended treatment method among treatment methods other than the medicine prescription for the patient. The recommended coping information is information for assisting the patient and, for example, is information that enables the patient side to cope with the pain even in a case where there is no doctor's intervention such as the examination by the doctor, by notifying at least one of the patient or the family member or the caregiver in advance of the information in a case where the pain is expected in the future.

The assistance information generation unit 58 may generate the pain treatment assistance information on a rule basis, for example, using a table indicating a correspondence relationship between the pain prediction information and the past pain pattern, and the pain treatment assistance information. The table may indicate the correspondence relationship between the pain prediction information and the past pain pattern, and the pain treatment assistance information.

Furthermore, the assistance information generation unit 58 can be configured by a model using a machine-learned algorithm and can use, for example, a support vector machine (SVM), a decision tree, a random forest, an adaboost, a neural network, or the like.

As described above, the control unit 51 can acquire the pain related information regarding the pain of the patient, input the acquired pain related information into the learning model 61 that outputs the pain prediction information in a case where the pain related information is input and acquire the pain prediction information of the patient, generate the pain treatment assistance information for the patient based on the acquired pain prediction information, and output the generated pain treatment assistance information.

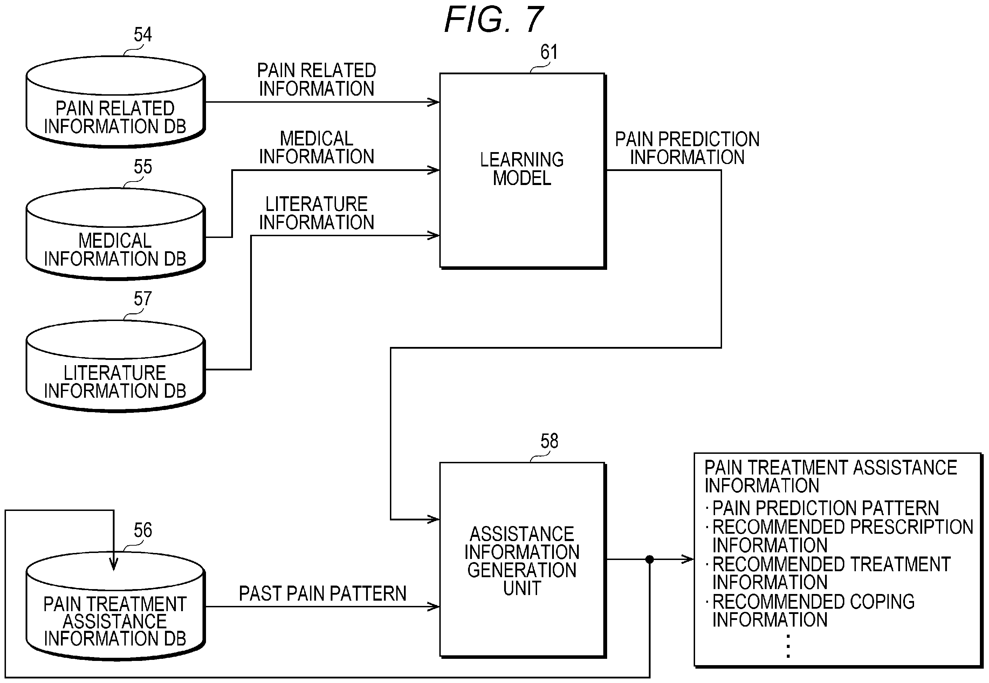

Fig. 7 is a diagram illustrating a second example of the processing for generating the pain treatment assistance information by the pain treatment assistance device 50. A difference from the first example illustrated in Fig. 6 is a point that the medical information and the literature information, in addition to the pain related information, are input into the learning model 61. The control unit 51 reads medical information of a specific patient from the medical information DB 55 and inputs the read medical information into the learning model 61. The medical information of the specific patient is time-series data at a single or a plurality of time points. For example, not only current medical information of the specific patient but also medical information at a past time point going back from the current time point may be included. The control unit 51 can perform the vectorization operation for generating the primary vector for the medical information, generate a medical information vector, and input the generated medical information vector into the learning model 61. Note that the medical information vector may be generated for each of the plurality of time points. The learning model 61 may, for example, output the pain prediction information from the present to one month later based on the pain related information for past three months and the medical information or may output the pain prediction information from the present to three month later, based on the pain related information for past six months and the medical information. Note that these periods are examples, and the present invention is not limited to these. Note that, although not illustrated, a past pain pattern of the pain treatment assistance information DB 56 may be input into the learning model 61.

Furthermore, the control unit 51 reads the literature information from the literature information DB 57 and inputs the read literature information into the learning model 61. The control unit 51 can perform the vectorization operation for generating the primary vector for the literature information, generate a literature information vector, and input the generated literature information vector into the learning model 61. It is sufficient that the literature information include, for example, information necessary for associating the type of history information such as the symptom history of the patient, the history of the vital data, the examination history, the medication history, or the treatment history, with how the situation of the pain of the patient changes in the future. For example, if there is a paper indicating how pain changes in the future when a specific medicine is taken, the paper can be used as the literature information. Furthermore, if there is a guideline indicating how the pain changes in the future when a specific treatment is performed, the guideline can be used as the literature information. In particular, by compensating shortage of the pain related information and the medical information with the literature information, in a case where the pain related information of the patient and the medical information are insufficient (for example, in a case where past data is small), the learning model 61 can output the pain prediction information of the patient. It is expected that accuracy of an index output by the learning model 61 is improved by increasing the number of types of information to be input into the learning model 61.

Furthermore, the control unit 51 reads the literature information from the literature information DB 57 and inputs the read literature information into the learning model 61. The control unit 51 can perform the vectorization operation for generating the primary vector for the literature information, generate a literature information vector, and input the generated literature information vector into the learning model 61. It is expected that accuracy of an index output by the learning model 61 is improved by increasing the number of types of information to be input into the learning model 61.

As described above, in a case of acquiring the medical information regarding the medical care of the patient and further inputting the medical information, the control unit 51 may input the acquired medical information into the learning model 61 that outputs the pain prediction information and acquire the pain prediction information of the patient.

Furthermore, in a case of acquiring the literature information regarding the pain treatment and further inputting the literature information regarding the pain treatment, the control unit 51 may input the acquired literature information into the learning model 61 that outputs the pain prediction information and acquire the pain prediction information of the patient.

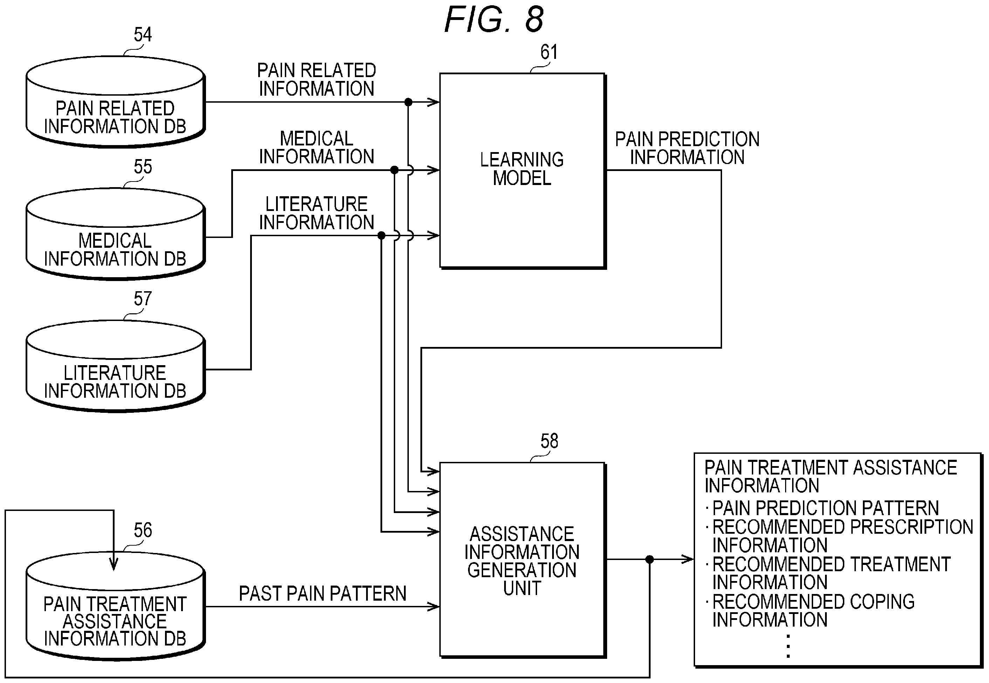

Fig. 8 is a diagram illustrating a third example of the processing for generating the pain treatment assistance information by the pain treatment assistance device 50. A difference from the second example illustrated in Fig. 7 is a point that the pain related information of the patient, the medical information, and the literature information are input into the assistance information generation unit 58, in addition to the pain prediction information and the past pain pattern. Note that, although not illustrated, a past pain pattern of the pain treatment assistance information DB 56 may be input into the learning model 61.

The assistance information generation unit 58 may generate the pain treatment assistance information on a rule basis, for example, using a table indicating a correspondence relationship between the pain prediction information, the past pain pattern, the pain related information of the patient, the medical information, and the literature information, and the pain treatment assistance information. The table may indicate the correspondence relationship between the pain prediction information, the past pain pattern, the pain related information of the patient, the medical information, and the literature information, and the pain treatment assistance information.

Furthermore, as in the first and second examples, the assistance information generation unit 58 can be configured by the model using the machine-learned algorithm and can use, for example, a support vector machine (SVM), a decision tree, a random forest, an adaboost, a neural network, or the like.

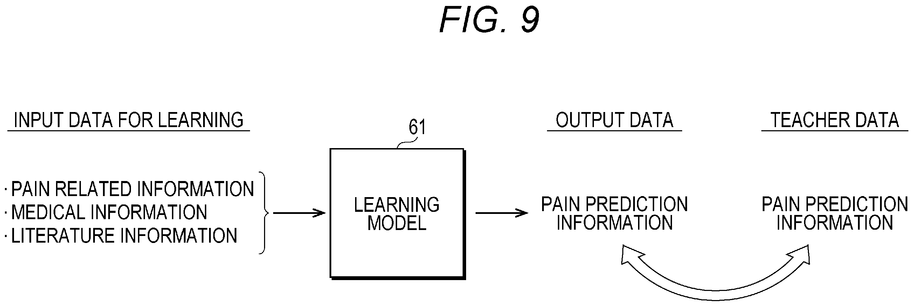

Fig. 9 is a diagram illustrating an example of a method for generating the learning model 61. Hereinafter, it is assumed that the pain treatment assistance device 50 generate the learning model 61. However, a learning processing device other than the pain treatment assistance device 50 may generate the learning model 61, and the pain treatment assistance device 50 may acquire the generated learning model 61. The control unit 51 acquires training data (second training data) including the pain related information regarding the pain of the plurality of patients, the medical information, the literature information regarding the pain treatment, and the pain prediction information of the plurality of patients. These pieces of training data include disease progress information and pain change information of the plurality of patients. The pain related information and the medical information are time-series data and include, for example, pain related information within a period of about one month, three months, six months, or 12 months in the past going back from the present. It is sufficient that the literature information include, for example, information necessary for associating the type of history information such as the symptom history of the patient, the history of the vital data, the examination history, the medication history, or the treatment history, with how the situation of the pain of the patient changes in the future. For example, if there is a paper indicating how pain changes in the future when a specific medicine is taken or when a specific treatment is performed, the paper can be used as the literature information. Furthermore, if there is a guideline indicating how the pain changes in the future when a specific treatment is performed, the guideline can be used as the literature information. The control unit 51 can generate the learning model 61 so as to output the pain prediction information in a case where the pain related information, the medical information, and the literature information regarding the pain treatment are input, based on the acquired training data. For example, the learning model 61 may be generated to output the pain prediction information from the present to one month later based on the pain related information for past three months and the medical information or may be generated to output the pain prediction information from the present to three month later, based on the pain related information for past six months and the medical information. At the time when the learning model 61 is generated, it is sufficient to adjust a parameter of the learning model 61 so that the pain prediction information output from the learning model 61 approaches the pain prediction information that is teacher data included in the training data.

Note that, in the example in Fig. 9, the pain related information, the medical information, and the literature information are included in the input data for learning. However, for example, it is not necessary to include at least one of the medical information and the literature information in the input data for learning. For example, in a case of acquiring training data (first training data) including the pain related information regarding the pain of the plurality of patients and the pain prediction information of the plurality of patients and inputting the pain related information based on the acquired training data, the control unit 51 may generate the learning model 61 so as to output the pain prediction information. However, it can be expected that inference accuracy of the learning model 61 is improved if at least one of the medical information and the literature information is included in the input data for learning.

Next, a specific example of the pain treatment assistance information will be described. The control unit 51 can output the pain treatment assistance information to the doctor terminal device 40, the patient terminal device 10, or the assistant terminal device 30. The pain treatment assistance information is displayed on the display panel of the doctor terminal device 40, and the doctor can perform an input operation or a selection operation according to displayed content. Similarly, the pain treatment assistance information is displayed on the display panel of the patient terminal device 10 or the assistant terminal device 30, and the patient, the family member or the caregiver, or the like can perform the input operation or the selection operation according to the displayed content.

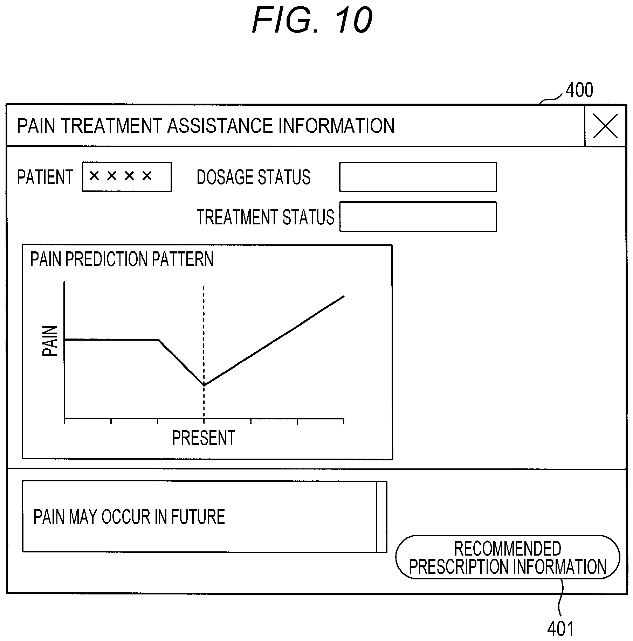

Fig. 10 is a diagram illustrating a first example of pain treatment assistance information 400 on a doctor side. In the pain treatment assistance information 400, patient information (patient ID, patient name, or the like), a dosage status (medicine type, dosage, medicine taking period, or the like), a treatment status (treatment method, treatment period, or the like), and the pain prediction pattern are displayed. In the example in Fig. 10, the pain prediction pattern is displayed as a line graph, and a transition of the pain of the patient from the past to the future can be seen. The doctor can read that, in the pain treatment of the patient up to the present, although the pain is once alleviated at the current time, it is expected that the pain gradually increases in the future.

Furthermore, as an advice (assistance information) to the doctor, "Pain may occur in future" is displayed. When the doctor operates a "recommended prescription information" icon 401, it is possible to display the recommended prescription information to be described later.

As described above, the control unit 51 outputs the pain prediction pattern of the patient to the doctor terminal device 40.

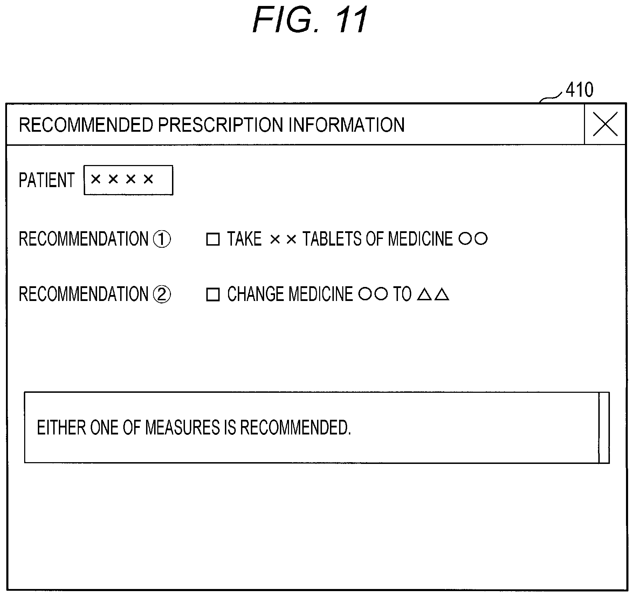

Fig. 11 is a diagram illustrating a second example of pain treatment assistance information 410 on the doctor side. The pain treatment assistance information 410 includes the recommended prescription information. In the recommended prescription information, recommended content is displayed together with the patient information. In the example in Fig. 11, as a recommendation 1, "Take XX tablets of medicine ○○" is displayed, and it is recommended to take a specific medicine. As a recommendation 2, "Change medicine ○○ to △△" is displayed, and a change of the medicine is recommended. Furthermore, in addition to the recommended content, "Either one of measures is recommended" is displayed, and this urges to adopt prescription recommended by the doctor. The recommended prescription information illustrated in Fig. 11 can be stored in the storage unit 59 as a table associated with a plurality of pain prediction patterns in advance. For example, pieces of recommended prescription information R1, R2, R3,... are associated with pain prediction patterns P1, P2, P3,.... The control unit 51 can select a pain prediction pattern closest to the pain prediction pattern of the patient as illustrated in Fig. 10 from the table and specify the recommended prescription information associated with the selected pain prediction pattern.

As described above, a pain occurrence frequency and period in the future can be predicted according to the pain prediction pattern of the patient, and the doctor can present analgesics suitable for the patient, in advance. When the patient takes the predicted analgesics, the pain predicted to occur in the future does not occur, and it is possible to improve quality of life (QOL) of the patient. In this way, it is possible to assist an appropriate pain treatment.

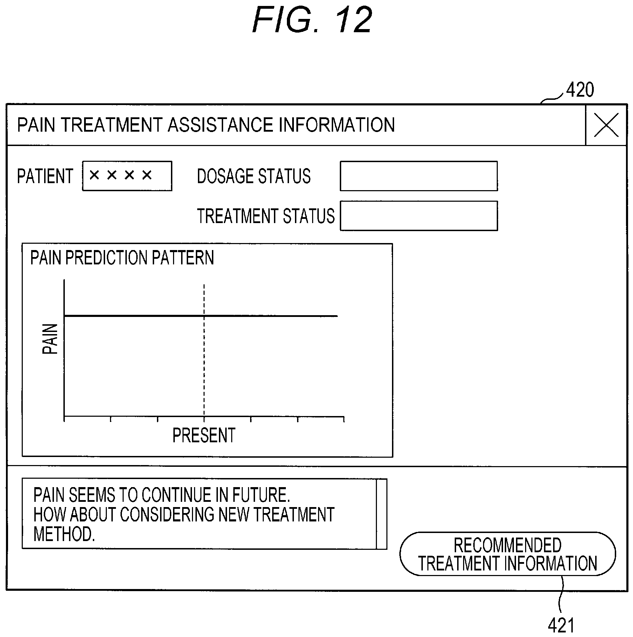

Fig. 12 is a diagram illustrating a third example of pain treatment assistance information 420 on the doctor side. As compared with the first example in Fig. 10, in the third example, pain transitions while the remains at a high value from the past to the present. The doctor can read that the pain treatment on the patient up to the present does not alleviate the pain.

Furthermore, as an advice (assistance information) to the doctor, "Pain seems to continue in future. How about considering new treatment method" is displayed. When the doctor operates a "recommended treatment information" icon 421, the recommended treatment information to be described later can be displayed.

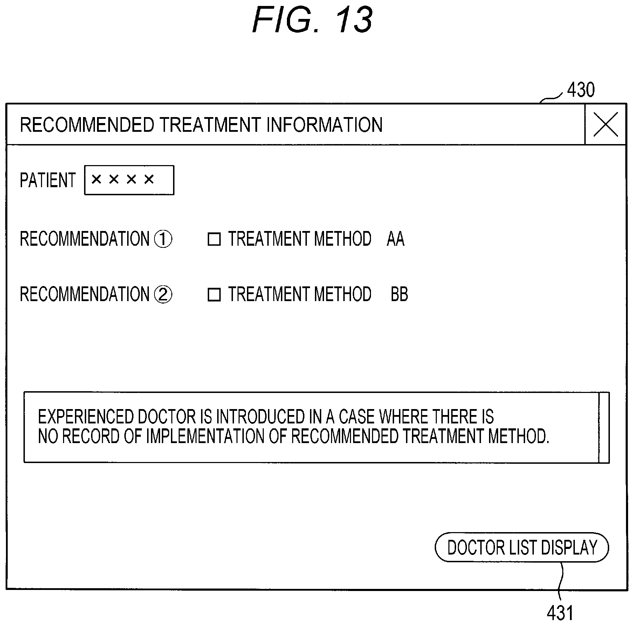

Fig. 13 is a diagram illustrating a fourth example of pain treatment assistance information 430 on the doctor side. The pain treatment assistance information 430 includes a recommended treatment method. In the recommended treatment method, recommended content is displayed together with the patient information. In the example in Fig. 13, as a recommendation 1, a treatment method AA is displayed, and as a recommendation 2, a treatment method BB is displayed. Furthermore, in addition to the recommended content, "Experienced doctor is introduced in a case where there is no record of implementation of recommended treatment method" is displayed. When the doctor operates a "doctor list display" icon 431, introduction information of doctors having experience in the recommended treatment method is displayed in a list format. The introduction information can include, for example, information such as a medical institution name, a name of a doctor, an experienced treatment method, or a contact address. Furthermore, it is also possible to contact a required doctor by operating a "contact" icon or the like. The recommended treatment method as illustrated in Fig. 13 can be stored in the storage unit 59 as a table associated with a plurality of pain prediction patterns in advance. For example, recommended treatment methods T1, T2, T3,... are associated with the pain prediction patterns P1, P2, P3,.... The control unit 51 can select a pain prediction pattern closest to the pain prediction pattern of the patient as illustrated in Fig. 12 from the table and specify the recommended treatment method associated with the selected pain prediction pattern.

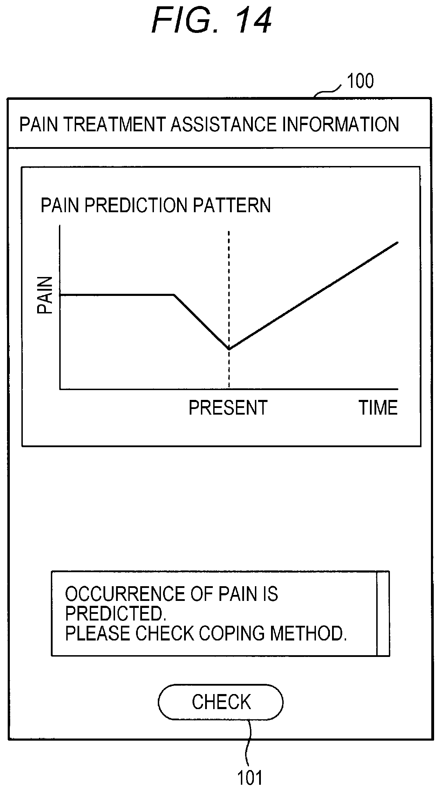

Fig. 14 is a diagram illustrating a first example of pain treatment assistance information 100 on the patient side. In the pain treatment assistance information 100, the pain prediction pattern is displayed. In the example in Fig. 14, the pain prediction pattern is displayed as a line graph, and a transition of the pain of the patient from the past to the future can be seen. The patient can read that, although the pain is once alleviated at the current time, it is expected that the pain gradually increases in the future.

Furthermore, as an advice (assistance information) to the patient or an assistant, "Occurrence of pain is predicted. Please check coping method" is displayed. When the patient or the assistant operates a "check" icon 101, a coping method to be described can be displayed.

As described above, the control unit 51 outputs the pain prediction pattern of the patient to the patient terminal device 10 and the assistant terminal device 30.

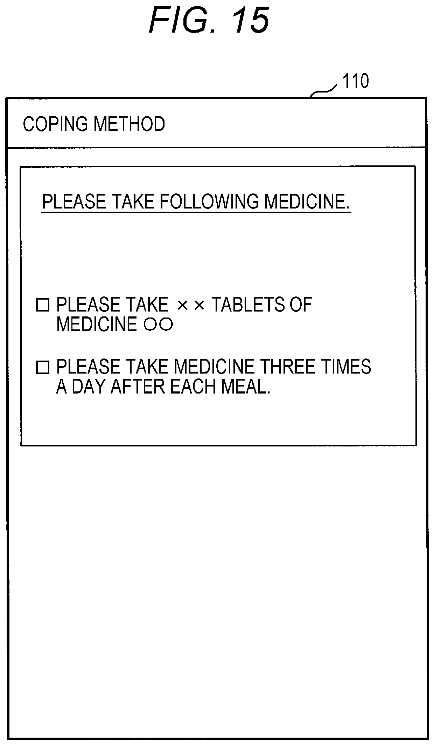

Fig. 15 is a diagram illustrating a second example of pain treatment assistance information 110 on the patient side. The pain treatment assistance information 110 includes the coping method (recommended coping information). In the example in Fig. 15, "Please take following medicine. Please take XX tablets of medicine ○○ three times a day after each meal" is displayed. The recommended coping information as illustrated in Fig. 15 can be stored in the storage unit 59 as a table associated with the plurality of pain prediction patterns in advance. For example, pieces of recommended prescription information S1, S2, S3,... are associated with the pain prediction patterns P1, P2, P3,.... The control unit 51 can select a pain prediction pattern closest to the pain prediction pattern of the patient as illustrated in Fig. 14 from the table and specify the recommended coping information associated with the selected pain prediction pattern. The doctor may determine, in advance, the pieces of recommended prescription information S1, S2, S3,... associated with the pain prediction patterns P1, P2, P3,....

As described above, according to the pain prediction pattern of the patient, the patient can know an occurrence frequency and period of the pain in the future, in advance. Then, by performing necessary coping methods, according to the coping method recommended by the pain treatment assistance device 50, it is possible to prevent the occurrence of the pain in advance, and the quality of life (QOL) of the patient can be improved. In particular, for example, the patient can perform the necessary coping method at home, without visiting the hospital. In this way, it is possible to assist an appropriate pain treatment. Note that, in a case where it is not preferable to directly notify the patient of the pain treatment assistance information on the patient side, it is sufficient to transmit the pain treatment assistance information to the assistant terminal device 30, instead of the patient terminal device 10.

As described above, according to the present embodiment, it is possible to predict pain that may occur in the patient in the future, from the state of the patient, and it is possible to perform the pain treatment for alleviating the pain on the patient in advance. Since preventive measures can be taken in this way, the pain of the patient can be reduced.

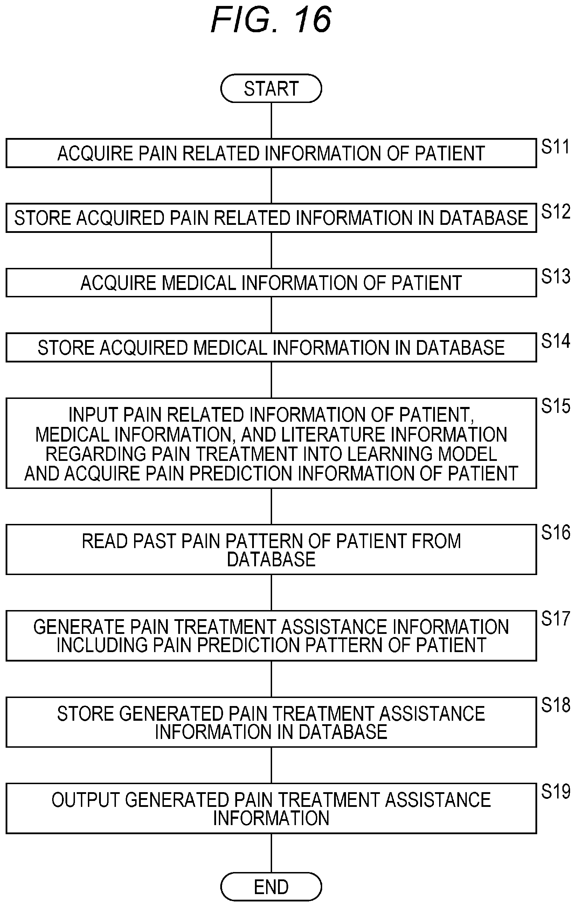

Fig. 16 is a diagram illustrating an example of a processing procedure by the pain treatment assistance device 50. Hereinafter, for convenience, the control unit 51 will be described as a subject of the processing. The control unit 51 acquires the pain related information of the patient (S11) and stores the acquired pain related information in the pain related information DB 54 (database) (S12). The pain related information can be repeatedly acquired at a required timing for each patient. For example, it can be acquired once or a plurality of times within a period from the time of the previous hospital visit to the time of the next hospital visit.

The control unit 51 acquires the medical information of the patient (S13) and stores the acquired medical information in the medical information DB 55 (database) (S14). The medical information can be acquired not only at the time of examination but also when the doctor determines that the medical information is needed.

The control unit 51 inputs the pain related information of the patient, the medical information, and the literature information regarding the pain treatment into the learning model 61 and acquires the pain prediction information of the patient, output by the learning model 61 (S15). The control unit 51 reads the past pain pattern of the patient from the pain treatment assistance information DB 56 (database) (S16).

The control unit 51 generates the pain treatment assistance information including the pain prediction pattern of the patient, based on the past pain pattern of the patient read from the pain treatment assistance information DB 56 and the pain prediction information of the patient acquired from the learning model 61 (S17). In this case, the pain treatment assistance information is generated based on the pain related information of the patient, the medical information, and the literature information, in addition to the past pain pattern and the pain prediction information.

The control unit 51 stores the generated pain treatment assistance information in the pain treatment assistance information DB 56 (database) (S18), outputs the generated pain treatment assistance information (S19), and ends the processing.

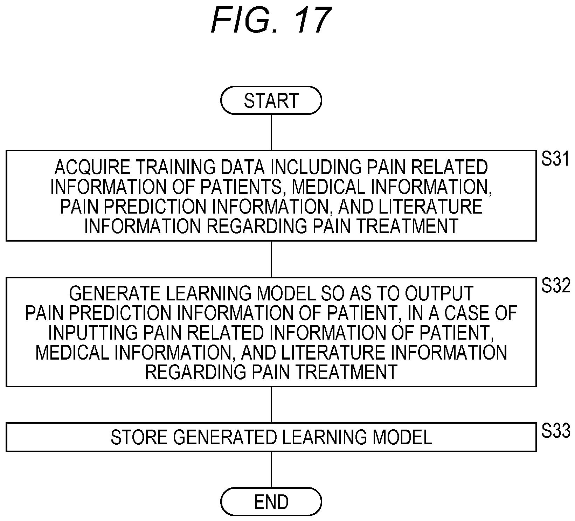

Fig. 17 is a diagram illustrating an example of a procedure for generating the learning model 61. The control unit 51 acquires the training data including the pain related information regarding the pain of the plurality of patients, the medical information, the pain prediction information, and the literature information regarding the pain treatment (S31). In a case of inputting the pain related information of each patient, the medical information, and the literature information regarding the pain treatment, based on the acquired training data, the control unit 51 generates the learning model 61 so as to output the pain prediction information of each patient (S32). The control unit 51 stores the generated learning model 61 in the storage unit 59 (S33) and ends the processing.

### Reference Signs List

- 1: communication network
- 10: patient terminal device
- 20: device
- 30: assistant terminal device
- 40: doctor terminal device
- 50: pain treatment assistance device
- 51: control unit
- 52: communication unit
- 53: memory
- 54: pain related information DB
- 55: medical information DB
- 56: pain management assistance information DB
- 57: literature information DB
- 58: assistance information generation unit
- 59: storage unit
- 60: computer program
- 61: learning model

## Claims

1. A computer program for causing a computer to execute processing comprising:
acquiring pain related information regarding pain of a patient;
in a case where the pain related information is input, inputting the acquired pain related information into a learning model that outputs pain prediction information and acquiring the pain prediction information of the patient;
generating pain treatment assistance information for the patient based on the acquired pain prediction information; and
outputting the generated pain treatment assistance information.

2. The computer program according to claim 1, wherein
the pain related information includes
at least one of an evaluation index used to evaluate pain, vital data, medication information, and a physical condition.

3. The computer program according to claim 1, for causing the computer to execute processing further comprising:
acquiring medical information regarding medical care of the patient; and
in a case where the medical information is further input, inputting the acquired medical information into the learning model that outputs the pain prediction information and acquiring the pain prediction information of the patient.

4. The computer program according to claim 3, wherein
the medical information includes
at least one of diagnosis information, examination information, treatment information, prescription information, and disease prognosis prediction information.

5. The computer program according to claim 1, for causing the computer to execute processing further comprising:
acquiring literature information regarding a pain treatment; and
in a case where the literature information regarding the pain treatment is further input, inputting the acquired literature information into the learning model that outputs the pain prediction information and acquiring the pain prediction information of the patient.

6. The computer program according to any one of claims 1 to 5, wherein
the pain treatment assistance information includes
at least one of recommended prescription information and recommended treatment information used to assist a doctor who examines the patient.

7. The computer program according to any one of claims 1 to 5, wherein
the pain treatment assistance information includes
a coping method for assisting the patient.

8. The computer program according to any one of claims 1 to 5, for causing the computer to execute processing further comprising:
outputting a pain prediction pattern indicating a temporal transition of a degree of pain based on the pain prediction information of the patient.

9. An information processing device comprising:
a first acquisition unit that acquires pain related information regarding pain of a patient;
a second acquisition unit that inputs the acquired pain related information into a learning model that outputs pain prediction information in a case where the pain related information is input and acquires the pain prediction information of the patient;
a generation unit that generates pain treatment assistance information for the patient based on the acquired pain prediction information; and
an output unit that outputs the generated pain treatment assistance information.

10. An information processing method comprising:
acquiring pain related information regarding pain of a patient;
in a case where the pain related information is input, inputting the acquired pain related information into a learning model that outputs pain prediction information and acquiring the pain prediction information of the patient;
generating pain treatment assistance information for the patient based on the acquired pain prediction information; and
outputting the generated pain treatment assistance information.

11. A learning model generation method comprising:
acquiring first training data including pain related information regarding pain of a plurality of patients and pain prediction information of the plurality of patients; and
generating a learning model so as to output the pain prediction information in a case where the pain related information is input, based on the acquired first training data.

12. The learning model generation method according to claim 11, further comprising:
acquiring second training data further including medical information of the plurality of patients, literature information regarding a pain treatment, and the pain prediction information of the plurality of patients; and
generating the learning model so as to output the pain prediction information, in a case where the medical information and the literature information regarding the pain treatment are input, based on the acquired second training data.
